# EUROPEAN PATENT APPLICATION

(11) **EP 4 552 678 A1**
(43) Date of publication of application: **14.05.2025**
(21) Application number: 24204486.5
(22) Date of filing: 03.10.2024
(51) Int. Cl.: A61M 25/00, A61M 27/00

(54) **CATHETER ASSEMBLY**

(30) Priority: 10.11.2023 SI 202300144
(71) Applicant: Tik d.o.o., 5222 Kobarid (SI)
(72) Inventor: Kurincic, Albert, 5222 Kobarid (SI); Kolenc, Sebastjan, 5222 Kobarid (SI); Zupancic, Martin, 1290 Grosuplje (SI)
(74) Representative: Golmajer Zima, Marjanca

(57) **Abstract**

A catheter assembly (100) comprising a catheter (1) with an insertable portion (2) and a non-insertable portion (3), and a packaging element (4) comprising a rigid tubular sleeve (5) to receive the catheter (1) and a wetting liquid, and a rigid cover sleeve (6) detachably fixed to an attachment (24) of the tubular sleeve, the non-insertable portion (3) comprising a tube portion (10) to receive the tube (2), said tube portion having on its outer periphery a section (32), the diameter of which is larger than the inner diameter of the attachment (24), such that, when the catheter (1) is inserted into the attachment (24) of the tubular sleeve, the tube portion (10) gets deformed by getting crinkled, thus forming a sealing spot (1) together with the inner surface (34) of the attachment, and a grip portion (11) for gripping the catheter (1) in use, which is provided with a conical through hole (31) and a free end (36); the cover sleeve (6) having an inner peripheral surface (33) and a circular rib (28) with a slanting surface (29) arranged at the base (27), the free end (36) of the grip portion tightly bearing on at least one inner surface (33, 29) of the cover sleeve to form a sealing spot (II).

## Description

### Prior art

Subjects experiencing problems with their bladder or rather its emptying often use a catheter. A catheter is a thin tube that is used to discharge urine from the bladder. A catheter has at least one opening at the end of the tube that is inserted into the bladder through the urethra, through which urine flows from the bladder into the catheter and out of the catheter at the other end, which is usually connected to a urine discharge attachment that the user directs into the toilet bowl or attaches it to a urine collection container. The user who uses the catheter and performs self-catheterisation uses it several times a day in different locations, not always in the home environment, wherein these types of catheters are most often for a single use. The basic requirement for the catheter is to have a smooth tube surface with adequate gliding properties to allow atraumatic insertion into the urethra without causing injury. The catheter is therefore made of plastics and is wetted either continuously or just before use. The catheter is stored in a packaging containing a liquid which wets the surface of the catheter before use, the packaging being configured in such a way to prevent leakage of the liquid which provides adequate gliding properties, such as distilled or sterilised water or gel, and thus allows the catheter to have an adequate shelf life, even for months or years. In addition to the functional requirements, the external appearance of the packaging is also important and should be unobtrusive, aesthetic and small enough to be stored in a handbag. Nowadays, plastic packaging is used for this type of catheter, either in a foil or rigid material. The catheter, especially the one for women, is more and more often stored in a rigid sleeve-shaped packaging, which is comparable to the packaging of any beauty aid and therefore inconspicuous.

EP 2635337 (Van Groningen et al) discloses a catheter assembly comprising a sleeve-shaped packaging and a catheter with an attachment housed therein. The sleeve consists of two parts and comprises a main body to receive the catheter and a cap closing the main body. In the main body of the sleeve, in the part closer to the opening, there is a flexible gel container provided with two openings in the longitudinal direction, through which the catheter tube passes. The catheter tube passes through the gel container and gets wet as the catheter is withdrawn from the sleeve. To prevent the gel from leaking out of the container into the rest of the sleeve and/or the cap, the diameters of the two openings are precisely defined and depend on the diameter of the catheter tube. The catheter has a specially shaped attachment that fits the flexible element. It is important that the gel container is inserted into the sleeve correctly and precisely, otherwise the gel will leak out of the container, either into the main body of the sleeve or into the cap. In both cases, the catheter does not get wet when withdrawn from the sleeve, which prevents or at least makes the insertion of the catheter into the urethra very difficult. If the gel leaks into the cap, it wets the part of the catheter that is intended to be grasped, which is uncomfortable for the user. The catheter assembly described is appropriate in the case where the catheter wetting liquid is a gel. If a lower viscosity liquid, such as distilled or sterilised water, is used for wetting, there is a high probability for the liquid to leak out of the container.

### Technical problem

The technical problem is to configure a catheter assembly comprised of a catheter and a rigid packaging that will allow using a low viscosity liquid, such as distilled or sterilised water, for catheter wetting, wherein the portion of the catheter intended for grasping must remain dry, while the portion of the catheter intended to be inserted into the urethra should be wetted and sterile. The catheter assembly must be such as to prevent leakage and/or volatilisation of the wetting liquid. At the same time, the catheter assembly must consist of a minimum number of components to make it simple and economical to manufacture. In addition, the catheter packaging must be configured to be aesthetic and inconspicuous.

### Solution to the technical problem

The technical problem is solved by a catheter assembly comprising a catheter including an insertable portion in the shape of a tube that is circularly closed at one end and provided with at least one opening for urine inflow and open at the other end, and a non-insertable portion arranged at the open end of the tube and provided in the longitudinal axis with a through hole of a varying diameter; and a packaging element comprising a rigid tubular sleeve that is closed at one end to receive the catheter and the wetting liquid, the other, open end, being formed as an attachment with an inner surface, and a rigid cover sleeve closed at one side by a base and closing the tubular sleeve and detachably fixed to the attachment of the tubular sleeve, the catheter being inserted with its insertable portion in the tubular sleeve that has been pre-filled with a wetting liquid, the catheter being covered by a rigid cover sleeve; the non-insertable portion comprising a tube portion to receive the tube, said tube portion having on its outer periphery a section, the diameter of which is larger than the inner diameter of the attachment, such that, when the catheter is inserted into the attachment of the tubular sleeve, the tube portion gets deformed by getting crinkled, thus forming a sealing spot I together with the inner surface of the attachment; and a grip portion for gripping the catheter in use, which provided with a conical through hole and a free end; the cover sleeve having an inner peripheral wall and a circular rib with a slanting surface arranged at the base, the free end of the grip portion tightly bearing on at least one inner surface of the cover sleeve to form a sealing spot II.

The grip portion of the non-insertable portion tightly bears on the circular rib of the cover sleeve with the inner surface of the conical hole. The cover sleeve has an inner peripheral surface that tightly bears on the outer peripheral surface of the tubular sleeve attachment and forms a sealing spot III. The outer periphery of the grip portion is formed unevenly in the form of ribs or other protrusions for better grip.

The tube portion has an outer periphery formed with a cone for easier insertion of the catheter into the tubular sleeve and with a limiting rib limiting the longitudinal displacement of the catheter within the tubular sleeve.

The packaging element is made of a plastic rigid material meeting the requirements for medical products. The non-insertable portion is made of a flexible plastic material.

The catheter assembly of the invention meets the fundamental requirements for a catheter - wettability, gliding properties and rounded edges for easier insertion of the catheter into the urethra, wherein the other, grip portion of the catheter, remains dry, and the wetting liquid is prevented from leaking and evaporating due to the sealing spots which are impermeable to low viscosity liquids, such as water, all this contributing to a long-term sterility and usability of the catheter assembly. As a result, it allows a carefree storing of the assembly, in a handbag for instance. The packaging element is made of a rigid plastic material, which can be shaped in any way, e.g. in the form of a sleeve, thus achieving a high degree of discretion. The advantage of the catheter assembly of the invention is also its simple production due to a low number of components, resulting in a potentially very economical production.

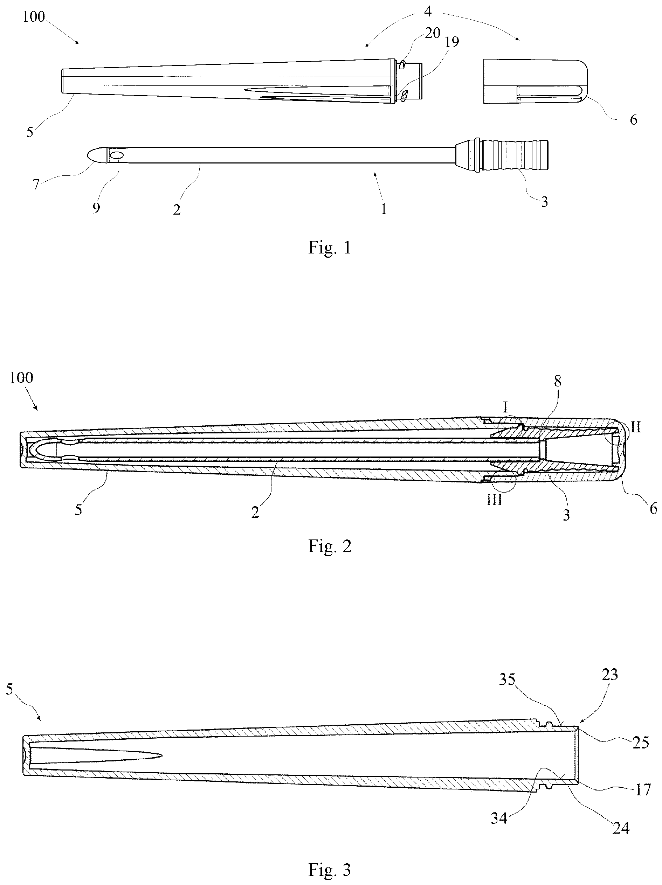

The catheter assembly of the invention will be described in more detail below by way of an embodiment and drawings representing in
Fig. 1 catheter assembly in exploded view
Fig. 2 catheter assembly in cross-section
Fig. 3 tubular sleeve in cross-section

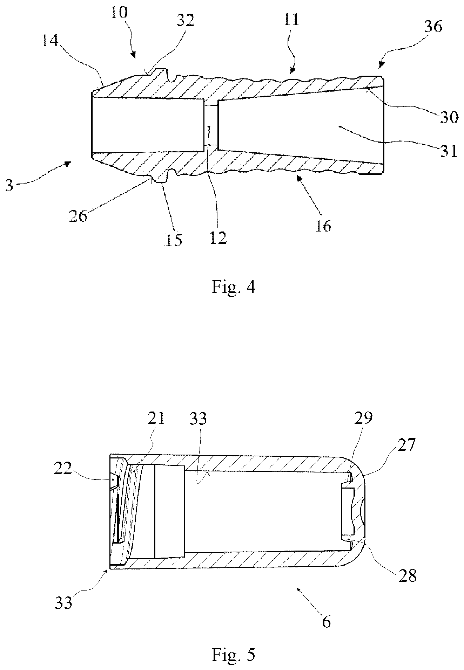

Fig. 4 non-insertable portion of the catheter in cross-section
Fig. 5 cover sleeve in cross-section

The catheter assembly comprises
- a catheter 1 comprising
   an insertable portion 2 in the shape of a tube insertable, when in use, into the urethra and intended to discharge urine from the bladder, and
   a non-insertable portion 3 arranged at an open end of the tube 2 and acting as a tube element when inserted in a packaging element 4 and as a grip element when the catheter 1 is in use, and
- a packaging element 4 comprising:
   a rigid tubular sleeve 5 intended to receive the catheter 1 and a wetting liquid,
   with which the catheter tube 2 is wetted, and
   a rigid cover sleeve 6 closing the tubular sleeve 5 and acting as a seal.

The packaging element 4 is made of any plastic rigid material meeting the requirements for medical products. A rigid material is a material that preserves its shape under normal operating conditions and protects the catheter 1 against damage when it is stored within the packaging element 4.

The catheter 1 comprises the insertable portion 2 and the non-insertable portion 3. The insertable portion 2 of the catheter in the shape of a tube is circularly closed at a first end 7 intended to be inserted into the urethra and has at least one opening 9 through which urine flows in when the catheter 1 reaches the female user bladder. At the opposite, other end 8, the insertable portion 2 is open and substantially adapted in its length to an average length of the urethra of female users. The non-insertable portion 3 of the catheter is arranged at the other end 8 of the tube. The insertable portion 3 is made of a flexible plastic material meeting the requirements for medical products and can get elastically deformed under the influence of an external force. The non-insertable portion 3 comprises a tube section 10 that receives the catheter tube 2, and a grip portion 11 intended to be gripped while the catheter 1 is used. The non-insertable portion 3 is provided with a through hole 12 of a varying diameter in its longitudinal axis. The diameter of the through hole 12 in the tube section 10 is such that the tube section 10 tightly bears on the tube 2 in the area of its other end 8. The through hole 12 is of conical shape in the grip portion 11, such that a conical hole 31 is formed that facilitates the discharge of urine and/or the attachment of the urine collection container.

The packaging element 4 comprises a rigid tubular sleeve 5 adapted in its length to the length of the catheter tube 2, and a rigid cover sleeve 6 that is normally shorter than the tubular sleeve 5 and covers the non-insertable catheter portion 3 when the catheter 1 is inserted in the tubular sleeve 5. The tubular sleeve 5 is closed at one end, while it is open at the opposite, other end 23, the other end 23 being formed as an attachment 24 with a threaded rib 19 and a lock pin 20 on the outer periphery. An inner edge 25 of the attachment is chamfered such that a slanted bearing surface 17 is formed, on which the tube portion 10 of the non-insertable portion bears with its slanted side 26 of the circumferential rib when the catheter 1 is inserted in the tubular sleeve 5. The cover sleeve 6 has two grooves 21, 22 arranged on the inner periphery, which are complementary to the threaded rib 19 and the lock pin 20 of the tubular sleeve and by means of which the cover sleeve 6 is detachably fixed to the attachment 24 and also to the tubular sleeve 5.

The catheter 1 is inserted in the tubular sleeve 5 with the non-insertable portion 2, the sleeve being pre-filled with a wetting liquid. The wetting liquid wets the outer surface of the insertable tubular portion 2 prior to use, while the non-insertable portion 3 projecting beyond the tubular sleeve 5, in particular the outer surface of the grip portion 11, must remain dry when the tubular sleeve 5 is closed by the cover sleeve 6. The packaging element 4 is divided into a wet zone and a dry zone, which is rendered possible by the sealing spots I, II, III which are formed between the tubular sleeve 5, the cover sleeve 6 and the non-insertable portion 3.

The tube portion 10 of the non-insertable portion has an outer periphery formed by a cone 14, which facilitates the insertion of the catheter 1 into the tubular sleeve 5, by a limiting rib 15 with a slanted side 26 facing the cone 14, and by a section 32 arranged therebetween, which is straight in this embodiment. The diameter of the section 32 is larger than the inner diameter of the attachment 24, such that when the catheter 1, specifically the tube portion 10, is inserted into the tubular sleeve 5, specifically the attachment 24, the tube portion 10 gets deformed by getting crinkled, thus creating the sealing spot I. The sealing spot I which is located between the inner peripheral surface 34 of the attachment and the section 32 of the tube portion 10 prevents the leakage of the wetting liquid between the tubular sleeve 5 and the non-insertable portion 3. When the catheter 1 is inserted into the tubular sleeve 5, the limiting rib 15 bears with its slanted side 26 onto the slanted bearing surface 17 of the attachment and limits the longitudinal displacement of the catheter 1 in the tubular sleeve 5.

The grip portion 11 has its outer periphery 16 formed unevenly in the form of ribs or other protrusions for better grip.

At the distal, i.e. free end, the catheter tube 2 has at least one opening 9 for the urine inflow into the catheter. A respective opening 9 allows the wetting liquid contained in the tubular sleeve 5 to flow into the tube 2 and further through the conical hole 31 of the non-insertable portion 3 to the cover sleeve 6, where the outer surface of the grip portion 11 is wetted. Wetting of the outer surface of the grip portion 11 is not desirable, this is why a second sealing spot II is formed between the grip portion 11 and the cover sleeve 6.

The cover sleeve 6 is formed as a short sleeve having a base 27. In the interior of the cover sleeve 6, on its base 27, a circular rib 28 is arranged. The wall 29 of the circular rib 28 facing the peripheral wall of the cover sleeve 6 is slanted. In the assembled configuration of the catheter assembly 100, the catheter 1 is inserted in the packaging element 4, the cover sleeve 6 being arranged and fixed to the tubular sleeve 5. The grip portion 11 of the non-insertable portion bears with its free end 36 on the inner surfaces of the cover sleeve 6. The grip portion 11 of the non-insertable portion tightly bears on the slanted wall 29 of the circular rib of the cover sleeve 6 with the inner surface 30 of the conical hole. A second sealing spot II is formed which prevents the leakage of the wetting liquid from the tube 2, i.e. the interior of the grip portion 11, into the space between the cover sleeve 6 and the grip portion 11. The outer surface of the grip portion 11 thus remains dry. In another, related embodiment, the grip portion 11 of the non-insertable portion bears with its outer circumferential surface on the inner circumferential surface 33 of the cover sleeve 6 such that a second sealing spot II is formed.

The cover sleeve 6 is detachably arranged on the attachment 24 of the tubular sleeve and thus on the tubular sleeve 5 by means of detachable connections threaded rib 19/complementary groove 21 and lock pin 20/complementary groove 22. When positioned, the cover sleeve 6 tightly bears with its inner circumferential surface 33 on the outer circumferential surface 35 of the attachment and a sealing spot III is formed. The sealing spot III is a further sealing spot which prevents the wetting liquid from leaking from the packaging element 4 in case that the wetting liquid leaks into the cover sleeve 6, thus providing for the functionality of the catheter 1.

## Claims

1. A catheter assembly (100) comprising
- a catheter (1) comprising
an insertable portion (2) in the shape of a tube that is circularly closed at a first end (7) and provided with at least one opening (9) for the inflow of urine, and open at the other end (8), and
a non-insertable portion (3) arranged at the open end (8) of the tube (2) and provided with a through hole (12) of a varying diameter in its longitudinal axis, and
- a packaging element (4) comprising
a rigid tubular sleeve (5) that is open at one end to receive the catheter (1) and the wetting liquid, while the other, open end (23) is formed as an attachment (24) with an inner (34) and an outer surface (35),
a rigid cover sleeve (6) that is closed at one side by a base (27), closes the tubular sleeve (5) and is detachably fixed to the attachment (24) of the tubular sleeve,
the catheter (1) being inserted with the non-insertable portion (2) in the tubular sleeve (5) which has been pre-filled with a wetting liquid, and the catheter (1) being covered by the rigid cover sleeve (6),
**characterized in that**
the non-insertable portion (3) comprises
a tube portion (10) to receive the tube (2), said tube portion having on its outer periphery a section (32) the diameter of which is larger than the inner diameter of the attachment (24) such that, when the catheter (1) is inserted into the attachment (24) of the tubular sleeve, the tube portion (10) gets deformed by getting crinkled, thus forming a first sealing spot (I) together with the inner surface (34) of the attachment,
and
a grip portion (11) for gripping the catheter (1) during use, provided with a conical through hole (31) and a free end (36),
the cover sleeve (6) has an inner peripheral surface (33) and a circular rib (28) with a slanting surface (29) arranged at the base (27),
the free end (36) of the grip portion tightly bearing on the at least one inner surface (33, 29) of the cover sleeve, such that a second sealing spot (II) is formed.

2. The catheter assembly according to claim 1, **characterized in that** the grip portion (11) of the non-insertable portion tightly bears on the circular rib (28) of the cover sleeve (6) with the inner surface (30) of the conical hole (31).

3. The catheter assembly according to any preceding claim, **characterized in that** the cover sleeve (6) has an inner peripheral surface (33) that tightly bears on the outer peripheral surface (35) of the attachment to form a third sealing spot (III).

4. The catheter assembly according to any preceding claim, **characterized in that** the tube portion (10) has the outer periphery formed with a cone (14) for the insertion of the catheter (1) into the tubular sleeve (5), and with a limiting rib (15).

5. The catheter assembly according to claim 1 or 2, **characterized in that** the grip portion (11) has its outer periphery (16) formed unevenly in the form of ribs or other protrusions for better grip.

6. The catheter assembly according to any preceding claim, **characterized in that** the non-insertable portion (3) is made of a flexible plastic material.

7. The catheter assembly according to any preceding claim, **characterized in that** the packaging element (4) is made of a plastic rigid material meeting the requirements for medical products.
